# EUROPEAN PATENT SPECIFICATION

(11) **EP 0 997 459 B1**
(45) Date of publication and mention of the grant of the patent: **26.11.2008**
(21) Application number: 00101297.0
(22) Date of filing: 04.11.1991
(51) Int. Cl.: C07D 277/24, A61K 31/425

(54) **Intermediates for preparing retroviral protease inhibiting compounds**
Zwischeprodukte zur Herstellung von retroviralen Protease-Hemmern
Intermédiaires pour la préparation des composés d'inhibiteurs de protéases rétrovirales

(30) Priority: 20.11.1990 US 616170; 15.08.1991 US 746020; 23.10.1991 US 777626
(43) Date of publication of application: 03.05.2000
(62) Divisional of application: 91119464.5
(73) Proprietor: ABBOTT LABORATORIES, Abbott Park, IL 60064 (US)
(72) Inventor: Kempf, Dale J., Libertyville, Illinois 60048 (US); Codacovi, Lynn M., Antioch, Illinois 60002 (US); Norbeck, Daniel W., Grayslake, Illinois 60030 (US); Plattner, Jacob J., Libertyville, Illinois 60048 (US); Sham, Hing L., Mundelein, Illinois 60060 (US); Wittenberger, Steven J., Mundelein, Illinois 60060 (US); Zhao, Chen, Libertyville, Illinois 60048 (US)
(74) Representative: Modiano, Micaela Nadia

(56) References cited:
- No relevant documents disclosed

## Description

### Technical Field

This invention was made with Government support under contract number AI27220 awarded by the National Institute of Allergy and Infectious Diseases. The Government has certain rights in this invention.

The present invention relates to novel intermediates employed in processes for malting compounds useful for inhibiting retroviral proteases and in particular for inhibiting human immunodeficiency virus (HIV) protease (in vitro, or in vivo. The retroviral protease inhibiting compounds are useful for treating a retroviral infection and in particular an HIV infection in a human or other mammal.

### Background Art

Retroviruses are those viruses which utilize a ribonucleic acid (RNA) intermediate and a RNA-dependent deoxyribonucleic acid (DNA) polymerase, reverse transcriptase, during their life cycle. Retroviruses include, but are not limited to, the RNA viruses of the Retroviridae family, and also the DNA viruses of the Hepadnavirus and Caulimovirus families. Retroviruses cause a variety of disease states in man, animals and plants. Some of the more important retroviruses from a pathological standpoint include human immunodeficiency viruses (HIV-1 and HIV-2), which cause acquired immune deficiency syndrome (AIDS) in man, hepatitis B virus, which causes hepatitis and hepatic carcinomas in man, human T-cell lymphotrophic viruses I, II, IV and V, which cause human acute cell leukemia, and bovine and feline leukemia viruses which cause leukemia in domestic animals.

Proteases are enzymes which cleave proteins at specific peptide bonds. Many biological functions are controlled or mediated by proteases and their complementary protease inhibitors. For example, the protease renin cleaves the peptide angiotensinogen to produce the peptide angiotensin I. Angiotensin I is further cleaved by the protease angiotensin converting enzyme (ACE) to form the hypotensive peptide angiotensin II. Inhibitors of renin and ACE are known to reduce high blood pressure in vivo. An inhibitor of a retroviral protease should provide a therapeutic agent for diseases caused by the retrovirus.

The genomes of retroviruses encode a protease that is responsible for the proteolytic processing of one or more polyprotein precursors such as the pol and gag gene products. See Wellink, Arch. Virol. 98 1 (1988). Retroviral proteases most commonly process the gag precursor into core proteins, and also process the pol precursor into reverse transciptase and retroviral protease. In addition, retroviral proteases are sequence specific. See Pearl, Nature 328 482 (1987).

The correct processing of the precursor polyproteins by the retroviral protease is necessary for the assembly of infectious virions. It has been shown that in vitro mutagenesis that produces protease-defective virus leads to the production of immature core forms which lack infectivity. See Crawford, J. Virol. 53 899 (1985); Katoh, et al., Virology 145 280 (1985). Therefore, retroviral protease inhibition provides an attractive target for antiviral therapy. See Mitsuya, Nature 325 775 (1987).

H.L. Sham et al., J. Chem. Soc. Chem. Commun., no. 2,15 January 1991, pp. 110-112 **disclose (2S, 5S, 4R)-2,5-Diamino-3,3-difluoro-1,6-diphenylhydroxyhexane as the core unit of a potent HIV proteinase inhibitor.**

Current-treatments for viral diseases usually involve administration of compounds that inhibit viral DNA synthesis. Current treatments for AIDS (Dagani, Chem. Eng. News, November 23, 1987 pp. 41-49) involve administration of compounds such as 2',3'-dideoxycytidine, 2',3'-dideoxyinosine, trisodium phosphonoformate, ammonium 21-tungsto-9-antimoniate, 1-beta-D-ribofuranosyl-1,2,4-triazole-3-carboxamide, 3'-azido-3'-deoxythymidine, and adriamycin that inhibit viral DNA synthesis; compounds such as AL-721 and polymannoacetate which may prevent HIV from penetrating the host cell; and compounds which treat the opportunistic infections caused by the immunosuppression resulting from HIV infection. None of the current AIDS treatments have proven to be totally effective in treating and/or reversing the disease. In addition, many of the compounds currently used to treat AIDS cause adverse side effects including low platelet count, renal toxicity and bone marrow cytopenia.

### Disclosure of the Invention

The present invention provides a compound of the formula: 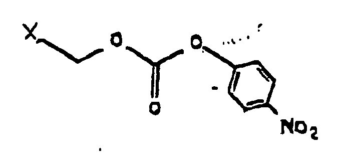 wherein X is thiazolyl. In a preferred embodiment X is 5-thiazolyl. In addition, the present invention provides a compound of the formula; 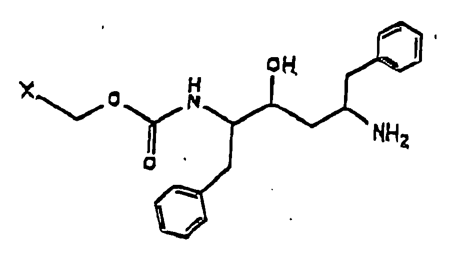 wherein X is thiazolyl, or an acid addition salt thereof. In a preferred embodiment, the compound is (2S, 3S, 5S)-5-amino-2-(N-(5-thiazolyl)-methoxycarbonyl)amino-1,6-diphenyl-3-hydroxyhexane; or an acid addition salt thereof.

The compounds of the invention are useful for preparing protease inhibiting compounds of the formula: 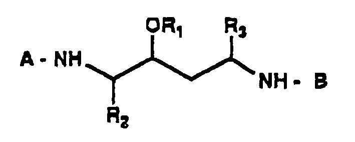 wherein R₁ is hydrogen,
R₂ and R3 are each benzyl,
A is -C(O)-R₆' wherein R₆' is (thiazolyl) methoxy and
B is R₆-C(O)-NH-CH(R₅ₐ)-C(O)- wherein R₅ₐ is loweralkyl and R₆ is R₇-R₉- wherein R₇ is heterocyclic or (heterocyclic) alkyl and R₉ is -N(R₇ₐ)-, S or O wherein R₇ₐ is hydrogen or loweralkyl;
or a pharmaceutically acceptable salt, prodrug or ester thereof.

The compounds of the invention comprise asymmetrically substituted centers. Such centers can be racemic or asymmetric. Racemic mixtures, mixtures of diastereomers, as well as single diastereomers of the compounds of the invention are included in the present invention. The terms "S" and "R" configuration are as defined by the IUPAC 1974 Recommendations for Section E, Fundamental Stereochemistry, Pure Appl. Chem. (1976) 45, 13 - 30.

The term "N-protecting group" or "N-protected" as used herein refers to those groups intended to protect the N-terminus of an amino acid or peptide or to protect an amino group against undersirable reactions during synthetic procedures. Commonly used N-protecting groups are disclosed in Greene, "Protective Groups In Organic Synthesis," (John Wiley & Sons, New York (1981)).

N-protecting groups comprise carbamates, amides, N-alkyl derivatives, amino acetal derivatives, N-benzyl derivatives, imine derivatives, enamine derivatives and N-heteroatom derivatives. Preferred N-protecting groups are formyl, acetyl, benzoyl, pivaloyl, t-butylacetyl, phenylsulfonyl, benzyl, t-butyloxyearbonyl (Boc), benzyloxycarbonyl (Cbz) and the like. N-protecting groups also refer to an L- or D-aminoacyl residue, which is derived from an L- or D- amino acid.

The term "O-protecting group" as used herein refers to a substituent which protects hydroxyl groups against undesirable reactions during synthetic procedures such as those O-protecting groups disclosed in Greene, "Protective Groups In Organic Synthesis," (John Wiley & Sons, New York (1981)). O-protecting groups comprise substituted methyl ethers, for example, methoxymethyl, benzyloxymethyl, 2-methoxyethoxymethyl, 2-(trimethylsilyl)ethoxymethyl, t-butyl, benzyl and triphenylmethyl; tetrahydropyranyl ethers; substituted ethyl ethers, for example, 2,2,2-trichloroethyl; silyl ethers, for example, trimethylsilyl, t-butyldimethylsilyl and t-butyldiphenylsilyl; and esters prepared by reacting the hydroxyl group with a carboxylic acid, for example, acetate, propionate, and benzoate,

The term "loweralkyl" as used herein refers to straight or branched chain alkyl radicals containing from 1 to 6' carbon atoms including, but not limited to, methyl, ethyl, n-propyl, iso-propyl, n-butyl, iso-butyl, sec-butyl, n-pentyl, 1-methylbutyl, 2,2-dimethylbutyl, 2-methylpentyl, 2,2-dimethylpropyl, and n-hexyl,

The term "aryl" as used herein refers to a C₆ monocyclic aromatic ring system or a C₉ or C₁₀ bicyclic carbocyclic ring system having one or more aromatic rings including, but not limited to, phenyl, naphthyl, tetrahydronaphthyl, indanyl, indenyl and the like. Aryl groups can be unsubstituted or substituted with one, two or three substituents independently selected from loweralkyl, haloalkyl, alkoxy, thioalkoxy, alkoxycarbonyl, alkanoyl, hydroxy, halo, mercapto, nitro, amino, alkylamino, dialkylamino, carboxaldehyde, carboxy, carboxamide, arylalkyl, arylalkoxy, (heterocyclic)alkyl', (heterocyclic) alkoxy, aminoalkyl, aminoalkoxy, alkylaminoalkyl, alkylaminoalkoxy, dialkylaminoalkyl, dialkylaminoalkoxy, (alkoxyalkyl)aminoalkyl, (alkoxyalkyl) aminoalkoxy, di-(alkoxyalkyl)aminoalkyl, di-(alkoxyalkyl) aminoalkoxy, (alkoxyalkyl) (alkyl) aminoalkyl, (alkoxyalkyl) (alkyl) aminoalkoxy, hydroxyalkyl, hydroxyalkoxy, carboxyalkyl, carboxyalkoxy, alkoxyalkyl, thioalkoxyalkyl, polyalkoxyalkyl and dialkoxyalkyl. In addition, substituted aryl groups include tetrafluorophenyl and pentafluorophenyl.

The term "arylalkyl" as used herein refers to an aryl group appended to a loweralkyl radical including, but not limited to, benzyl, 4-hydroxybenzyl and 1-naphthylmethyl.

The term "aminoalkyl" as used herein refers to -NH₂ appended to a loweralkyl radical.

The term "hydroxyalkyl" as used herein refers to -OH appended to a loweralkyl radical.

The term "alkylamino," as used herein refers to a loweralkyl radical appended to an NH radical.

The term "cycloalkyl" as used herein refers to an aliphatic ring having 3 to 7 carbon atoms including, but not limited to, cyclopropyl, cyclopentyl, cyclohexyl and the like. Cycloalkyl groups can be unsubstituted or substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, carboalkoxy and carboxamide.

The term "cycloalkylalkyl" as used herein refers to a cycloalkyl group appended to a loweralkyl radical, including but not limited to cyclohexylmethyl.

The terms "alkoxy" and "thioalkoxy" as used herein refer to R₂₉O- and R₂₉S-, respectively, wherein R₂₉ is a loweralkyl group or benzyl.

The term "alkoxyalkyl" as used herein refers to an alkoxy group appended to a loweralkyl radical.

The term "thioalkoxyalkyl" as used herein refers to a thioalkoxy group appended to a loweralkyl radical.

The term "hydroxyalkoxy" as used herein refers to -OH appended to an alkoxy radical. The term "(heterocyclic) alkoxy" as used herein refers to R₃₄O- wherein R₃₄ is a (heterocyclic) alkyl group.

The term "dialkylamino" as used herein refers to -NR₃₆R₃₇ wherein R₃₆ and R₃₇ are independently selected from loweralkyl groups.

The term "alkylaminoalkyl" as used herein refers to NHR₄₁ appended to a loweralkyl radical, wherein R₄₁ is a loweralkyl group.

The term "dialkylaminoalkyl" as used herein refers to NR₄₄ R₄₅ which is appended to a loweralkyl radical wherein R₄₄ and R₄₅ are independently selected from loweralkyl.

The term "carboxyalkyl" as used herein refers to a carboxylic acid group (-COOH) appended to a loweralkyl radical.

The term "polyalkoxyalkyl" as used herein refers to a polyalkoxy residue appended to a loweralkyl radical.

The term "polyalkoxy" as used herein refers to -OR₆₇ wherein R₆₇ is a straight or branched chain containing 1-5, C_{n'}-O-C_{n"} linkages wherein n' and n" are independently selected from 1 to 3, including but not limited to methoxyethoxymethoxy, methoxymethoxy and the like.

The term "alkoxyalkyl (alkyl) amino" as used herein refers to -NR₈₅R₈₆ wherein R₈₅ is an alkoxyalkyl group and R₈₆ is a loweralkyl group. The term "di-(alkoxyalkyl) amino" as used herein refers to -NR₈₇R₈₈ wherein R₈₇ and R₈₈ are alkoxyalkyl groups.

The term "halo" or "halogen" as used herein refers to - Cl, -Br, -I or -F.

The term "haloalkyl" as used herein refers to a loweralkyl radical in which one or more of the hydrogen atoms are replaced by halogen including, but not limited to, chloromethyl, trifluoromethyl, 1-chloro-2-fluoroethyl and the like.

The term "thioalkoxyalkyl" as used herein refers to a thioalkoxy group appended to a loweralkyl radical.

The term "alkanoyl" as used herein refers to Rₖ-C(O)-wherein Rₖ is a loweralkyl group.

The term "aminoalkoxy" as used herein refers to an alkoxy radical to which is appended an amino (-NH₂) group.

The term "alkylaminoalkoxy" as used herein refers to an alkoxy radical to which is appended an alkylamino group

The term "dialkylaminoalkoxy" as used herein refers to an alkoxy radical to which is appended a dialkylamino group.

The term "(alkoxyalkyl) aminoalkyl" refers to a loweralkyl radical to which is appended an (alkoxyalkyl)amino group. The term "(alkoxyalkyl) aminoalkoxy" as used herein refers to an alkoxy radical to which is appended an (alkoxyalkyl)amino group.

The term "(alkoxyalkyl) (alkyl) aminoalkyl" refers to a loweralkyl radical to which is appended an (alkoxyalkyl) (alkyl) amino group.

The term "(alkoxyalkyl) (alkyl) aminoalkoxy" as used herein refers to an alkoxy radical to which is appended an (alkoxyalkyl) (alkyl) amino group.

The term "di- (alkoxyalkyl) aminoalkyl" refers to a loweralkyl radical to which is appended an di-(alkoxyalkyl) amino group.

The term "d-(alkoxyalkyl) aminoalkoxy" as used herein refers to an alkoxy radical to which is appended an di-(alkoxyalkyl) amino group.

The term "carboxyalkoxy" as used herein refers to an alkoxy radical to which is appended a carboxy (-COOH) group.

At each occurrence, the term "heterocyclic ring" or "heterocyclic" as used herein independently refers to a 3- or 4-membered ring containing a heteroatom selected from oxygen, nitrogen and sulfur; or a 5- or 6-membered ring containing one, two or three heteroatoms independently selected from N, O and S. The 5-membered ring has 0-2 double bonds and the 6-membered ring has 0-3 double bonds. The nitrogen heteroatoms can be optionally quaternized or N-oxidized. The sulfur heteroatoms can be optionally S-oxidized. The term "heterocyclic also includes bicyclic groups in which any of the above heterocyclic rings is fused to a benzene ring or a cyclohexane ring or another heterocyclic ring. Heterocyclics include: pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolinyl, oxazolidinyl, isoxazolyl, isoxazolinyl, isoxazolidinyl, morpholinyl, thiazolyl, thiazolidinyl, isothiazolyl, isothiazolidinyl, indolyl, quinolinyl, tetrahydroquinolyl, isoquinolinyl, benzimidazolyl, benzothiazolyl, benzoxazolyl, benzofuranyl, furyl, dihydrofuranyl, tetrahydrofuranyl, pyranyl, dihydropyranyl, tetrahydropyranyl, dioxanyl, dioxolanyl, thienyl and benzothienyl.

Heterocyclics also include:

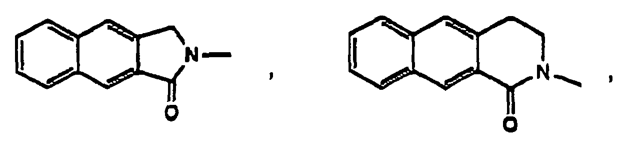

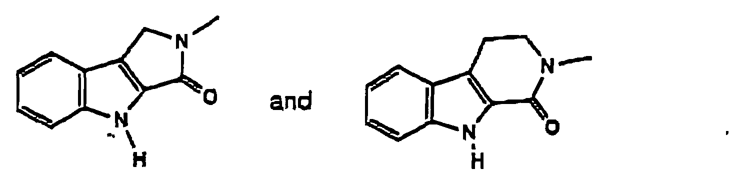

Heterocyclics can be unsubstituted or monosubstituted or disubstituted with substituents independently selected from hydroxy, halo, oxo (=O), alkylimino (R*N= wherein R* is a loweralkyl group), amino, (N-protected)amino, alkylamino, (N-protected) alkylamino, dialkylamino, alkoxy, polyalkoxy, haloalkyl, cycloalkyl, aryl, arylalkyl, -COOH, -SO₃H and loweralkyl. In addition, nitrogen containing heterocycles can be N-protected.

The term "(heterocyclic) alkyl" as used herein refers to a heterocyclic group appended to a loweralkyl radical, including but not limited to imidazolylmethyl and thiazolylmethyl.

The term "(alkoxyalkyl) aminoalkyl" as used herein refers to a loweralkyl radical to which is appended R₃₁₁NH- wherein R₃₁₁ is an alkoxyalkyl group

When any variable group occurs more than one time in any substituent or in a compound, its definition on each occurrence is independent of its definition at every other occurrence. Also, combinations of substituents and/or variables are permissible only if such combinations result in stable compounds.

Compounds useful as intermediates for the preparation of the compound of formula XXXVII in Scheme 3 are the compounds of the formula: 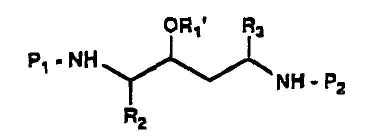 wherein P₁ and P₂ are independently selected from hydrogen and an N-protecting group; R₁' is hydrogen or an O-protecting group; and R₂ and R₃ are each benzyl; or a salt thereof.

Intermediates and products can be prepared as shown in Schemes 1-3. The syntheses of carboxylic acids (A-OH and B-OH) and p-nitrophenyl esters (A-OPNP and B-OPNP) are described in the Examples. The process shown in Scheme 1 discloses the pinacol coupling of a protected aminoaldehyde (I) to give (II) and (III). Diols (II) and (III) are independently deoxygenated by initial reaction with α-acetoxyisobutyryl bromide and lithium bromide followed by reduction of the intermediate bromoacetate with tri-n-butyltin hydride to provide (IV) and (V), respectively. Basic hydrolysis of (IV) and (V) leads to (VI) and (VII), respectively.

Scheme 2 illustrates the preparation of a particular substituent A which is N-(N'-2-pyridylmethyl-N'-methylaminocarbonyl)-L-valine (XXXV), which is a particular substituent prepared in Example 3. 2-Picolinaldehyde (XXXI) is converted to 2-(N-methyl)aminomethylpyridine (XXXII) by treatment with methylamine, followed by hydrogenation. Reaction of (XXXII) with the methyl or benzyl ester of N-phenoxycarbonyl-L-valine ((XXXIII) provides (XXXIV). Hydrolysis (R=Me) or hydrogenation (R=benzyl) of (XXXIV) provides (XXXV).

Scheme 3 illustrates the preparation of compounds of wherein A and B are not identical. Starting with diamine (XXXVII), monoacylation of the diamine with the p-nitrophenyl carbonate of A-OH provides a mixture of (XXXVIII) and (XXXIX). This mixture can be separated by silica gel chromatography. Acylation of (XXXVIII) with the p-nitrophenyl ester or p-nitrophenyl carbonate of B-OH provides (XL). 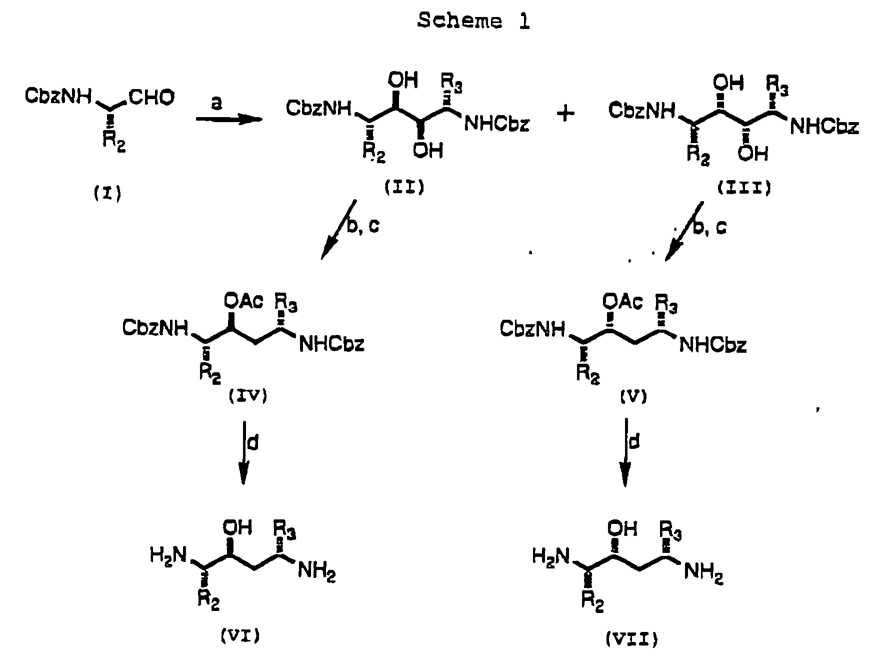 a, VCl₃·(THF)₃, Zn, CH₂Cl₂; b, LiBr, α-acetexyisobutyryl bromide, CH₃CN; c, (n-Bu)₃SnH, AIBN, THF; d, Ba(OH)₂· 8H₂O, H₂O, dioxane. R₂ and R₃ are each benzyl. 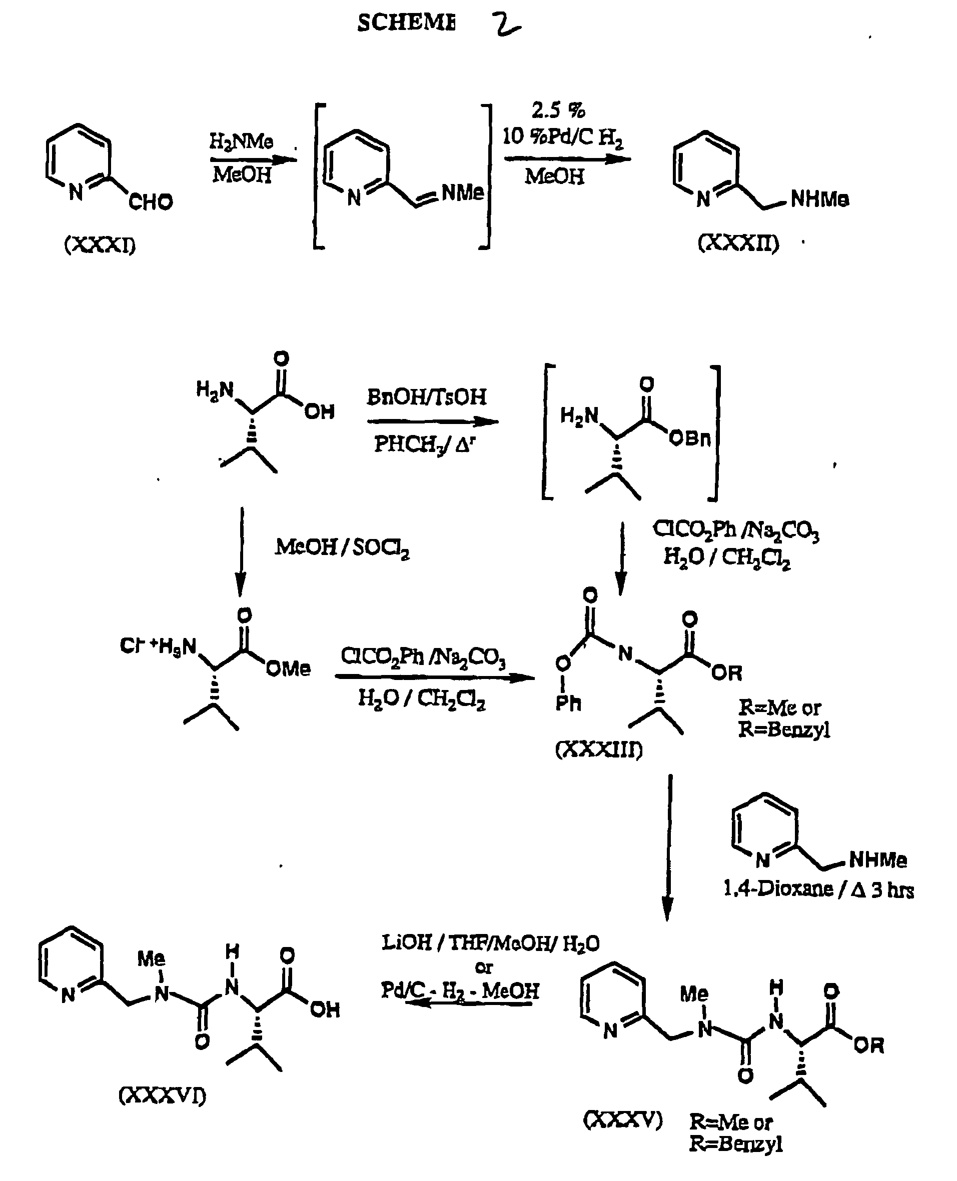 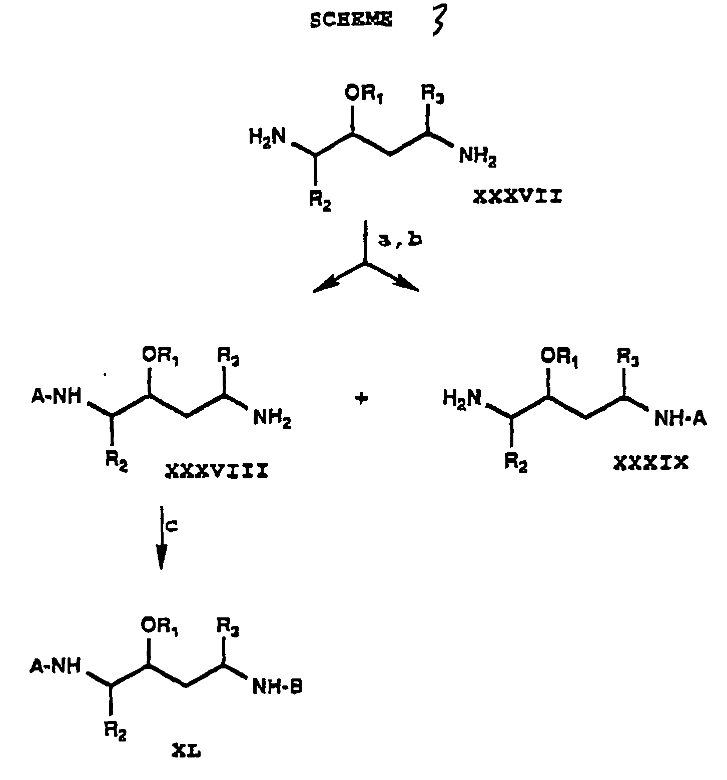 a, A-OPNP, THF or CH₂Cl₂; b, silica gel chromatography; c B-OPNP, THF or CH₂Cl₂. R₁ is hydrogen and R₂ and R₃ are each benzyl.

The following examples will serve to further illustrate preparation of the novel compounds of the invention. The compound of Example 7C and the compound of Example 8 used in Example 9 are compounds of the invention.

### Example 1

### A. Cbz-L-phenylaninal.

A solution of 24.5 ml of anhydrous dimethyl sulfoxide in 870 ml of anhydrous dichloromethane was cooled under N₂ atmosphere to -60°C and treated over a period of 15 min with 131 ml of a 2 M solution of oxalyl chloride in dichloromethane in order that the internal temperature remained below -50°C. After addition, the solution was stirred at -60°C for 15 min and treated over a period of 20 min with a solution of 50 g (0.175 mol) of Cbz-L-phenylalaninol in 200 ml of dichloromethane. The resulting solution was stirred at -60°C for 1 h, then treated over a period of 15 min with 97 ml of triethylamine in order that the internal temperature remained below -50°C. After addition the solution was stirred at -60°C for 15 min, then, with the cooling bath in place, was treated rapidly (over a period of 1 min) with a solution of 163 g of citric acid in 550 ml of water. The resulting slurry was stirred vigorously for 10 min, allowed to warm, diluted to 1 liter with water, and separated. The organic layer was washed with 700 ml of water followed by a mixture of 550 ml of water and 150 ml of saturated aqueous NaHCO₃, dried over MgSO₄, and concentrated in vacuo at 20°C to give the crude desired compound as a light yellow solid.

### B. (2S, 3R, 4R, 5S)-2,5-Bis-(N-Cbz-amino)-3,4-dihydroxy-1,6-diphenylhexane and (2S, 3S, 4S, 5S)-2,5-Bis-(N-Cbz-amino)-3,4-dihydroxy-1,6-diphenylhexane.

A suspension of 78.5 g of VCl₃· (tetrahydrofuran)₃ and 16 g of zinc dust in 400 ml of dry dichloromethane was stirred under N₂ atmosphere for 1 h at 25°C. A solution of 0.175 mol of Cbz-L-phenylalaninal in 200 ml of dichloromethane was then added in one portion, and the resulting mixture was stirred at ambient temperature under N₂ atmosphere for 16 h. The resulting mixture was added to 500 ml of 1 M aqueous HCl, diluted with 500 ml of hot chloroform, and shaked vigorously for 2 min. The layers were separated, and the organic layer was washed with 1 M aqueous HCl and separated. Filtration of the organic phase provided the crude desired product as a solid residue. The residue was slurried in 1.25 liters of acetone, treated with 5 ml of concentrated H₂SO₄, and stirred for 16 h at ambient temperature. The resulting mixture was filtered, and the residue (residue A) was washed with 50 ml of acetone. The combined filtrate was concentrated to a volume of 250 ml, diluted with 1000 ml of dichloromethane, washed three times with water and once with saturated brine, dried over MgSO₄, and concentrated to give a viscous oil. The oil was taken up in 1000 ml of 1 M HCl in methanol (prepared from 71 ml of acetyl chloride and 1000 ml of methanol) and stirred at ambient temperature for 2 h. The resulting precipitate was filtered, washed with methanol, and air-dried on the filter to provide 26.7 g of the desired compound as a white solid. The filtrate was concentrated and filtered to give a second crop (8.3 g) of (2S,3R,4R,5S)-2,5-bis-(N-Cbz-amino)-3,4-dihydroxy-1,6-diphenylhexane. ¹H NMR (d₆-DMSO) δ 2.59 (dd, J = 13, 5 Hz, 2H), 2.74 (dd, J = 13, 9 Hz, 2H), 3.26 (br, 2H), 4.19 (m, 2H), 4.54 (m, 2H), 4.92 (m, 4H), 6.82 (d, J = 9Hz, 2H), 7.0-7.35 (m, 20H). Mass spectrum: (M + H)⁺ = 569.

Residue A (above, 2.65 g) was suspended in 75 ml of tetrahydrofuran and 75 ml of 1 M aqueous HCl and heated at reflux for 24 h. After concentration of the resulting solution in vacuo, the residue was taken up in 10% methanol in chloroform, washed two times with water, dried over Na₂SO₄ and concentrated in vacuo to provide (2S, 3S, 4S, 5S)-2,5-bis-(N-Cbz-amino)-3,4-dihydroxy-1,6-diphenylhexane as a white solid. ¹H NMR (d₆-DMSO) δ 2.64 (m, 2H), 3.04 (m, 2H), 3.49 (m, 2H), 3.78 (m, 2H), 4.70 (d, J = 7 Hz, 2H), 4.93 (AA', 4H), 7.1-7.4 (m, 20H). Mass spectrum: (M + H)⁺ = 569.

### C. (2S,3R,4S,5S)-3-Acetoxy-2,5-bis-(N-Cbz-amino)-3-bromo-1,6-diphenylhexane.

A suspension of 25 g (44 mmol) of (2S,3R,4R,5S)-2,5-bis-(N-Cbz-amino)-3,4-dihydroxy-1,6-diphenylhexane in 500 ml of 2:1 dichloromethane/hexane was treated with 23 g of α-acetcxyisobutyryl bromide. The resulting mixture was stirred at ambient temperature until the reaction clarified, washed with two 200 ml portions of saturated aqueous NaHCO₃, dried over MgSO₄, and concentrated in vacuo to give 30.8 g of the crude desired compound. A portion was purified by silica gel chromatography using 9:1 dichloromethane:ethyl acetate to provide the pure desired compound as a white solid. ¹H NMR (CDCl₃) δ 2.21 (s, 3 H), 2.62 (dd, J = 13, 11 Hz, 1 H), 2.75 (d, J = 7 Hz, 2 H), 2.95 (br d, J = 15 Hz, 1 H), 4.03 (br t, J = 10 Hz, 1h), 4.40 (br d, J = 10Hz, 1 H), 4.6-5.0 (m, 6 H), 5.12 (br d, J = 13 Hz, 1 H), 5.33 (br d, J = 11 Hz, 1 H), 7.0-7.4 (m, 10 H). Mass spectrum: (M + NH₄)⁺ = 690, 692.

### D (2S,3S,5S)-3-Acetoxy-2,5-bis-(N-Cbz-amino)-1,6-diphenylhexane.

A solution of 30.8 g (44 mmol) of the crude resultant compound of Example 1C in 600 ml of tetrahydrofuran was treated with 17.8.ml (66 mmol) of tri-n-butyltin hydride and 1.45 g (8.8 mmol) of 2,2'-azobis-[2-methylpropionitrile]. The resulting solution was heated at reflux under N₂ atmosphere for 1.5 h. After being allowed to cool, the solution was concentrated in vacuo, and the residue was taken up into acetonitrile and washed with four portions of hexane. The acetonitrile layer was dried over MgSO₄, filtered, and concentrated in vacuo to provide 32 g of the crude desired compound. Mass spectrum: (M + NH₄)⁺ = 612.

### E. (2S,3S,5S)-2,5-Diamino-1,6-diphenyl-3-hydroxyhexane.

A suspension of 32 g of the crude resultant compound of Example 1D and 55.5 g (176 mmol) of barium hydroxide octahydrate in 400 ml of 1,4-dioxane and 400 ml of water was heated at reflux for 4 h. The resulting mixture was filtered, and the residue was rinsed with dioxane. The combined filtrates were concentrated to a volume of approximately 200 ml and extracted with four 400 ml portions of chloroform. The combined organic layers were dried over Na₂SO₄, filtered, and concentrated in vacuo. The residue was purified by silica gel chromatography using first 2% isopropylamine in chloroform and then 2% isopropylamine/2% methanol in chloroform to provide 10.1 g (81%) of the pure desired compound as a white solid. ¹H NMR (CDCl₃) δ 1.54 (dt, J = 14, 10 Hz, 1H), 1.67 (dt, J = 14, 3 Hz, 1H), 2.50 (dd, J = 13, 8Hz, 1H), 2.58 (dd, J = 13, 8Hz, 1H), 2.8 (m, 2 H), 2.91 (dd, J = 13, 5 Hz, 1 H), 3.10 (m, 1H),-3.72 (ddd, J = 11, 3, 2 Hz, 1H), 7.1-7.4 (m, 10H). Mass spectrum: (M + H)⁺ = 285.

### Example 2

### A. α-Isocyanato-valine Methyl Ester.

A suspension of L-valine methyl ester hydrochloride (49 g, 0.29 mol) in toluene (700 ml) was heated to 100°C and phosgene gas was bubbled into the reaction mixture. After approximately 6 h, the mixture became homogeneous. The bubbling of phosgene was continued for 10 more min, then the solution was cooled with the bubbling of N₂ gas. The solvent was then evaporated and the residue chased with toluene two times. Evaporation of solvent gave 40.8 g (89%) of the crude desired compound.

### B. N-((2-Pyridinyl)methoxycarbonyl)-valine Methyl Ester.

A solution of 0.78 g (5.0 mmol) of the resultant compound of Example 2A and 0.55 ml (5.7 mmol) of pyridine-2-methanol in 30 mL of toluene was heated at reflux under N₂ atmosphere for 4 h. The solvent was removed in vacuo, and the residue was purified by silica gel chromatography using 2% methanol in chloroform to give 0.72 g (54%) of the desired compound as an oil. ¹H NMR (CDCl₃) δ 0.91 (d, J = 7 Hz, 3H), 0.98 (d, J = 7 Hz, 3H), 2.19 (m, 1H), 3.75 (s, 3H), 4.32 (dd, J = 9, 5 Hz, 1H), 5.24 (s, 2H), 5.39 (br d, 1H), 7.23 (ddd, J = 8, 4, 1Hz, 1 H), 7.37 (d, J = 8 Hz, 1H), 7.70 (td, J = 8, 2 Hz, 1H), 8.60 (br d, 1H). Mass spectrum: (M + H) ⁺ = 267.

### C. N-((2-Pyridinyl)methoxycarbonyl)-valine.

Using the procedure of Example 3E but replacing the resultant compound of Example 3D with the resultant compound of Example 2B provided the desired compound.

### D . N-((2-Pyridinyl)methoxycarbonyl)-valine p-Nitrophenyl Ester.

Using the procedure of Example 3F but replacing the resultant compound of Example 3E with the resultant compound of Example 2C provided the desired compound.

### E. (2S, 3S, 5S)-2,5-Bis-(N-(N-((2-pyridinyl)methoxycarbonyl)-valinyl)-amino)-1,6-diphenyl-3-hydroxyhexane.

A solution of 0.13 g (0.46 mmol) of the resultant compound of Example 1E in 2 ml of dry dimethylformamide was treated with 0.5 g of the resultant compound of Example 2D. After being stirred at ambient temperature for 16 h, the solution was treated with saturated aqueous NaHCO₃, extracted with 5% methanol in chloroform, dried over Na₂SO₄, and concentrated in vacuo. The residue was purified by silica gel chromatography using a gradient of 2% - 3% - 5% methanol in chloroform to provide 161 mg (45%) of the pure desired compound, m.p. 220-222°C. Mass spectrum: (M + H)⁺ = 753.

Anal. Calcd for C₄₂H₅₂N₆O₇·0.5H₂O: C, 66.21; H, 7.01; N, 11.03. Found: C, 65.92; H, 6.90; N, 10.80.

### Example 3

### A. 2-(N-(t-Butyloxycarbonyl)aminomethyl)pyridine.

A solution of 21.2 g (97 mmol) of di-t-butyldicarbonate in 200 ml of dichloromethane was cooled to 0°C and treated in portions with 10 ml (97 mmol) of 2-(aminomethyl)pyridine. After being allowed to warm to ambient temperature and stirred overnight, the resulting solution was diluted with 100 ml of dichloromethane, washed with three 100 ml portions of water, dried over Na₂SO₄, and concentrated in vacuo to provide 19.8 g (98%) of the desired compound (R_{f} 0.28, 5% methanol in chloroform). ¹H NMR (CDCl₃) δ 1.47 (s, 9H), 4.45 (d, J = 6 Hz, 2H), 5.56 (br, 1H), 7.18 (m, 1H), 7.28 (d, J = 8 Hz, 1H), 7.66 (td, J = 7, 2Hz, 1H), 8.53 (m, 1H). Mass spectrum: (M + H)⁺ = 209.

### B. 2-((N-(t-Butyloxycarbonyl)-N-methylamino)methyl)pyridine.

A solution of 19.8 g (95 mmol) of the resultant compound of Example 3A in anhydrous tetrahydrofuran was cooled under N₂ atmosphere to 0°C and treated with 4.95 g (124 mmol) of sodium hydride (60% dispersion in oil). The solution was stirred for 15 min, treated dropwise with 7.1 ml (114 mmol) of methyl iodide, stirred at ambient temperature for 2 h, and quenched cautiously with water. The resulting mixture was partitioned between ether and water, dried over Na₂SO₄, and concentrated in vacuo. Chromatography on silica gel provided 14.9 g (70%) of the desired compound as a colorless oil. ¹H NMR (CDCl₃) δ 1.43, 1.49 (two s, 9H), 2.89, 2.94 (two s, 3 H), 4.54, 4.57 (two s, 2H), 7.2 (m, 2H), 7.67 (td, J = 8, 2 Hz, 1H), 8.55 (d, J = 4Hz, 1H). Mass spectrum: (M + H)⁺ = 223.

### C. 2-(N-Methylamino)methyl)pyridine Dihydrochloride.

The resultant compound of Example 3B (10 g) was treated with 200 ml of 6 M aqueous HCl and heated at reflux for 10 min. After being allowed to cool, the solution was concentrated in vacuo. The residue was treated twice with 50 ml of dioxane and concentrated in vacuo to provide the crude desired compound as a light brown solid.

### D. N-((N-Methyl-N-((2-pyridinyl)methyl)amino)carbonyl)-valine Methyl Ester.

A mixture of 1.61 g (7.2 mmol) of the resultant compound of Example 3C and 1.14 g (7.2 mmol) of the resultant compound of Example 2A in 40 ml of dichloromethane was treated with 2 ml (18 mmol) of 4-methylmorpholine. After being stirred for 2 h, the solution was partitioned between dichloromethane and water, dried over Na₂SO₄, and concentrated. Chromatography on silica gel using 2% methanol in chloroform provided 1.94 g (96%) of the desired compound (R_{f} 0.32, 5% methanol in chloroform) as a colorless oil. ¹H NMR (CDCl₃) δ 0.93 (d, J = 7 Hz, 3H), 0.97 (d, J = 7Hz, 3H), 2.16 (m, 1H), 3.03 (s, 3H), 3.72 (s, 3H), 4.43 (dd, J = 8, 5Hz, 1H), 4.55 (s, 2 H), 6.15 (br, 1H), 7.22 (dd, J = 8, 6Hz, 1H), 7.28 (d, J = 6 Hz, 1H), 7.69 (br t, 1H), 8.55 (d, J = 5Hz, 1H). Mass spectrum: (M + H)⁺ = 280.

### E. N-((N-Methyl-N-((2-pyridinyl)-methyl)-amino)-carbonyl)-valine.

A solution of 4.47 g (16 mmol) of the resultant compound of Example 3D in 65 ml of dioxane was treated with 65 ml of 0.5 M aqueous lithium hydroxide. After being stirred at ambient temperature for 1 h, the resulting solution was concentrated in vacuo to a small volume (ca. 5 ml), neutralized to pH 5 with 1 M aqueous HCl, and extracted with three 100 ml portions of ethyl acetate. The combined organic layers were dried over Na₂SO₄ and concentrated in vacuo to provide 3.61 g (85%) of the desired compound as an oil.

### F. N-((N-Methyl-N-((2-pyridinyl)-methyl)-amino)-carbonyl)-valine p-Nitrophenyl Ester.

A solution of 3.61 g (13.6 mmol) of the resultant compound of Example 3E and 2.3 g (16 mmol) of p-nitrophenol in 60 ml of anhydrous tetrahydrofuran was treated with 3.09 g (15 mmol) of dicyclohexyl carbodiimide and stirred under N₂ atmosphere at ambient temperature for 4 h. The resulting mixture was filtered and the residue was rinsed with fresh tetrahydrofuran. The combined filtrates were concentrated in vacuo to provide the crude desired compound as a yellow oil.

### G. (2S, 3S, 5S)-2,5-Bis-(N-(N-((N-methyl-N-((2-pyridinyl)-methyl)-amino)-carbonyl)-valinyl)-amino)-1,6-diphenyl-3-hydroxyhexane.

A solution of 1.26 g (4.44 mmol) of the resultant compound of Example 1E in 20 ml of 1:1 tetrahydrofuran: dimethylformamide was treated with 11 mmol of the resultant compound of Example 3F. After being stirred at ambient temperature under N₂ atmosphere for 16 h, the resulting solution was diluted with 600 ml of ethyl acetate, washed with five 200 ml portions of aqueous NaHCO_{3,} dried over Na₂SO₄, and concentrated in vacuo. Purification of the residue on silica gel using first 2% methanol in chloroform then 5% methanol in chloroform provided 2.95 g (86%) of the pure desired compound as a white solid, m.p. 134-137°C. Mass spectrum: (M + H)⁺ = 779.

Anal. Calcd for C₄₄E₅₈N₈O₅·1.5H₂O: C, 65.57; H, 7.63; N, 13.90. Found: C, 65.74; H, 7.24; N, 13.83.

### Example 4

### A. α-Isocyanato-isoleucine Methyl Ester.

Using the procedure of Example 2A but replacing L-valine methyl ester hydrochloride with L-isoleucine methyl ester hydrochloride provided the desired compound as an oil.

### B. N-((N-Methyl-N-((2-pyridinyl)methyl)amino)carbonyl)-isoleucine Methyl Ester.

Using the procedure of Example 3D but replacing the resultant compound of Example 2A with the resultant compound of Example 4A provided the desired compound. ¹H NMR (CDCl₃) δ 0.92 (t, J=7 Hz, 3 H), 0.94 (d, J = 7Hz, 3 H), 1.21 (m, 1 H), 1.46 (m, 1 H), 1.90 (m, 1 H), 3.02 (s, 3 H), 3.71 (s, 3 H), 4.46 (dd, J = 8, 5Hz, 1 H), 4.53 (s, 2 H), 6.15 (br, 1 H), 7.22 (dd, J = 7, 5Hz, 1 H), 7.27 (d, J = 7 Hz, 1 H), 7.69 (td, J = 7, 2 Hz, 1 H), 8.55 (br d, 1 H).

### C. N-((N-Methyl-N-((2-pyridinyl)methyl)amino)carbonyl)-isoleucine p-Nitrophenyl Ester.

Using the procedures of Example 3E and Example 3F with the resultant compound of Example 4 provided the crude desired compound.

### D. (2S, 3S, 5S)-2,5-Bis-(N-(N-((N-methyl-N-((2-pyridinyl)-methyl)amino)carbonyl)isoleucinyl)amino)-1,6-diphenyl-3-hydroxyhexane.

Using the procedure of Example 3G but replacing the resultant compound of Example 3F with the resultant compound of Example 4 provided, after silica gel chromatography using 2% methanol in chloroform, the desired compound in 68% yield. The pure compounds melted at 143-145°C, resolidified, and melted again at 173-174°C. Mass spectrum; (M + H)⁺ = 807.

### Example 5

### A. ((3-Pyridinyl)methyl)-(4-nitrophenyl)carbonate.

A solution 20 g (0.1 mol) of (4-nitrophenyl)-chloroformate in 150 ml of dichloromethane was cooled to 0°C and treated sequentially with 8.0 ml (0.083 mol) of pyridine-3-methanol and 11 ml (0.1 mol) of 4-methylmorpholine. After addition, the solution was allowed to come to ambient temperature, stirred for 0.5 h, diluted with dichloromethane, washed sequentially with aqueous NaHCO₃ and water, dried over Na₂SO₄, and concentrated in vacuo. The residue was broken up, triturated with 3;1 hexane:ethyl acetate, and filtered. The resulting solid was dissolved in a minimum amount of boiling ethyl acetate/hexane, filtered hot to remove an insoluble dark oil, and allowed to cool. The desired crystalline product (18.65 g, 82%) was collected by filtration.

### B. (2S, 3S, 5S)-5-Amino-2-(N-((3-pyridinyl)methoxycarbonyl)amino)-1,6-diphenyl-3-hydroxyhexane and (2S,3S,5S)-2-Amino-5-(N-((3-pyridinyl)methoxycarbonyl)-amino)-1,6-diphenyl-3-hydroxyhexane.

A solution of 1.5 g (5.28 mmol) of the resultant compound of Example 1E in 10 ml of tetrahydrofuran was treated dropwise over a 5 hour period with a solution of 1.6 g (5.8 mmol) of the resultant compound of Example 5A in 10 ml of tetrahydrofuran. After addition, the resulting solution was stirred at ambient temperature for 16 h and concentrated in vacuo. Silica gel chromatography using a gradient of 2-3.5% methanol in chloroform provided a mixture of the two desired compounds. Silica gel chromatography of the mixture using first 2% isopropylamine in dichloromethane followed by 2% isopropylamine/2% methanol in dichloromethane provided 0.38 g (16%) of (2S,3S,5S)-5-amino-2-(N-((3-pyridinyl)methoxycarbonyl)amino)-1,6-diphenyl-3-hydroxyhexane and 0.87 g (36%) of (2S, 3S, 5S)-2-amino-5-(N-((3-pyridinyl)methoxycarbonyl)amino)-1,6-diphenyl-3-hydroxyhexane.

### C. (2S, 3S, 5S)-2-(N-(N-((N-Methyl-N-((2-pyridinyl)-methyl)amino)carbonyl)valinyl)amino)-5-(N-((3-pyridinyl)-methoxycarbonyl)amino)-1,6-diphenyl-3-hydroxyhexane.

A solution of 1.2 g (2.86 mmol) of (2S, 3S, 5S)-2-amino-5-(N-((3-pyridinyl)methoxycarbonyl)amino)-1,6-diphenyl-3-hydroxyhexane in 20 ml of tetrahydrofuran was treated with 1.55 g (4.01 mmol) of the resultant compound of Example 3F. The resulting solution was stirred at ambient temperature for 96 h, treated with aqueous NaHCO₃, extracted with chloroform, dried over Na₂SO₄, and concentrated in vacuo. The residued was purified by silica gel chromatography using first 2% then 4% methanol in chloroform to provide 1.75 g (92%) of the ' desired compound (R_{f} 0.28, 10% methanol in chloroform) as a white solid, m.p. 69-71°C. Mass spectrum: (M + 1)⁺ = 667.

Anal. Calcd for C₃₈H₄₆N₆O₅·0.5H₂O: C, 67.54; H, 7.01; N, 12.44. Found: C, 67.54; H, 6.83; N, 12.33.

### Example 6

### ((2-Thiazolyl)methyl}-(4-nitrophenyl)carbonate.

A solution 2.3 g (11.5 mmol) of (4-nitrophenyl)-chloroformate in 20 ml of dichloromethane was cooled to 0°C and treated sequentially with a solution of 1.2 g (10.4 mmol) of 2-(hydroxymethyl) thiazole (Dondoni, et. al., Synthesis, 1987, 998; Tetrahedron Lett. 1983, 24, 2901) in 5 ml of dichloromethane and 1.7 ml (15.7 mmol) of 4-methylmorpholine. After addition, the solution was allowed to come to ambient temperature, stirred for 0.5 h, and concentrated in vacuo. Silica gel chromatography of the residue using first chloroform then 1% methanol in chloroform provided 1.15 g (39%) of the desired compound (R_{f} 0.73, 10% methanol in chloroform).

### Example 7

### A. 5-(Carbethoxy)thiazole.

According to the procedure of Mashraqui and Keehn (J. Am. Chem. Soc. 1982, 104, 4461), ethyl α-chloro-α-formylacetate was condensed with thioformamide and vacuum distilled to provide 5.65 g (33%) of the desired compound.

### B. 5-(Hydroxymethyl)thiazole

According to the procedure of Mashraqui and Keehn (J. Am. Chem. Soc. 1982, 104, 4461), 5-(carbethoxy) thiazole was reduced with lithium aluminum hydride to provide the crude desired compound in 44% yield.

### C. ((5-Thiazolyl)methyl)-(4-nitrophenyl) carbonate.

Using the procedure of Example 6 but replacing 2-(hydroxymethyl)thiazole with 5-(hydroxymethyl)thiazole and allowing the reaction to proceed at ambient temperature for 2 days provided, after silica gel chromatography using 6% ethyl acetate in chloroform, 1.1 g (71%) of the desired compound (R_{f} 0.22, 6% ethyl acetate in chloroform). Mass spectrum: (M + 1)⁺ = 281.

### Example 8

### A. (2S, 3S, 5S)-2-Amino-5-(N-((5-thiazolyl)-methoxycarbonyl)amino)-1,6-diphenyl-3-hydroxyhexane and (2S, 3S, 5S)-5-Amino-2-(N-((5-thiazolyl)-methoxycarbonyl)amino)-1,6-diphenyl-3-hydroxyhexane.

Using the procedure of Example 5B but replacing the resultant compound of Example 5A with the resultant compound of Example 7C provided, after silica gel chromatography using first 2% then 5% methanol in chloroform provided a mixture of the two desired compounds. Silica gel chromatography of the mixture using first 2% isopropylamine in chloroform followed by 2% isopropylamine/1% methanol in chloroform and finally 2% isopropylamine/2% methanol in chloroform provided 111 mg (16%) of (2S,3S,5S)-2-amino-5-(N-((5-thiazolyl)-methoxycarbonyl)amino)-1,6-diphenyl-3-hydroxyhexane and 185 mg (28%) of (2S, 3S, 5S)-2-amino-5-(N-((5-thiazolyl)-methoxycarbonyl)amino)-1,6-diphenyl-3-hydroxyhexane. Mass spectrum for each compound: (M + 1)⁺ = 426.

### Example 9

### (2S, 3S, 5S)-5-(N-(N-((N-Methyl-N-((2-pyridinyl)methyl)-amino)carbonyl)isoleucinyl)amino)-2-(N-((5-thiazolyl)-metoxycarbonyl)amino)-1,6-diphenyl-3-hydroxyhexane.

Using the procedure of Example 5C but replacing (2S, 3S, 5S)-2-amino-5-(N-((3-pyridinyl)methoxycarbonyl)-amino)-1,6-diphenyl-3-hydroxyhexane with (2S, 3S, 5S)-5-amino-2-(N-((5-thiazolyl)methoxycarbonyl)amino)-1,6-diphenyl-3-hydroxyhexane and replacing the resultant compound of Example 3F with the resultant compound of Example 4C provided, after silica gel chromatography using a gradient of 2% - 5% methanol in chloroform, 51 mg (62%) of the desired compound (R_{f} 0.47, 10% methanol in chloroform) as a white solid, m.p. 64-67°C. Mass spectrum: (M + 1) ⁺ = 687.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

Preferred salts of the compounds of the invention include hydrochloride, methanesulfonate, sulfonate, phosphonate and isethionate.

## Claims

1. A compound of the formula: 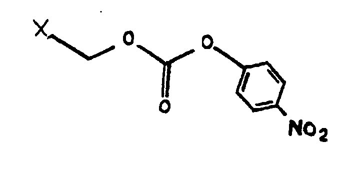 wherein X is thiazolyl.

2. The compound of Claim 1 wherein X is 5-thiazolyl.

3. A compound of the formula: 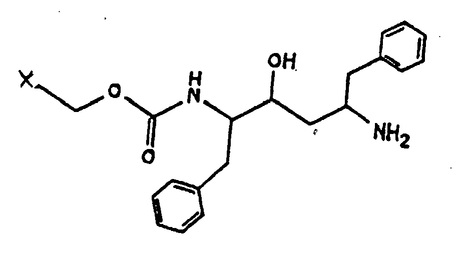 wherein X is thiazolyl, or an acid addition salt thereof.

4. The compound of Claim 3 which is (2S,3S,5S)-5-amino-2-(N-(5-thiazolyl)-methoxycarbonyl)amino-1,6-diphenyl-3-hydroxyhexane; or an acid addition salt thereof.

## Patentansprüche

1. Eine Verbindung mit der Formel: 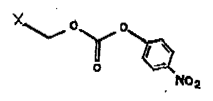 worin X Thiazolyl ist.

2. Die Verbindung von Anspruch 1, worin X 5-Thiazolyl ist.

3. Eine Verbindung mit der Formel: 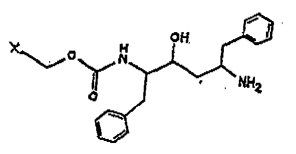 worin X Thiazolyl ist oder ein Säureadditionssalz davon.

4. Die Verbindung von Anspruch 3, die (2S, 3S, 5S)-5-Amino-2-(N-(5-thiazolyl)-methoxycarbonyl)amino-1,6-diphenyl-3-hydroxyhexan ist; oder ein Säureadditionssalz davon.

## Revendications

1. Composé de formule 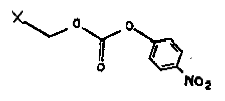 dans laquelle X est thiazolyle.

2. Composé selon la revendication 1, dans laquelle X est 5-thiazolyle.

3. Composé de formule: 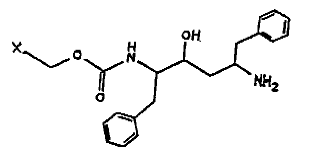 dans laquelle X est thiazolyle, ou un de ses sels d'addition d'acides.

4. Composé selon la revendication 3, qui est le (2S, 3S, 5S)-5-amino-2-(N-(5-thiazolyl)méthoxycarbonyl)amino-1,6-diphényl-3-hydroxyhexane; ou un de ses sels d'addition d'acides.
